# FASCICULE DE BREVET EUROPEEN

(11) **EP 0 971 854 B1**
(45) Date de publication et mention de la délivrance du brevet: **29.11.2006**
(21) Numéro de dépôt: 99903554.6
(22) Date de dépôt: 03.02.1999
(51) Int. Cl.: C01B 21/086, C01B 21/097, C07C 211/63, C07C 211/64, C07C 381/12, C07D 213/20, C07D 213/26, C07D 233/58, C07F 9/54, C08F 136/14, H01M 10/40, H01M 6/16

(54) **NOUVEAUX MATERIAUX UTILES EN TANT QUE SOLUTES ELECTROLYTIQUES**
NEUE ALS ELEKTROLYTISCHE SOLUBILISATE GEEIGNETE WERKSTOFFE
NOVEL MATERIALS USEFUL AS ELECTROLYTIC SOLUTES

(30) Priorité: 03.02.1998 CA 2228801; 18.12.1998 CA 2256945
(43) Date de publication de la demande: 19.01.2000
(60) Demande divisionnaire: 05023466.5
(73) Titulaire: ACEP INC., Montreal, Quebec H2Z 1A4 (CA); CENTRE NATIONAL DE LA RECHERCHE SCIENTIFIQUE (CNRS), 75794 Paris Cedex 16 (FR); Université de Montréal, Montréal, Québec H3C 3J7 (CA)
(72) Inventeur: MICHOT, Christophe, F-38000 Grenoble (FR); ARMAND, Michel, Montréal, Québec H3T 1N2 (CA); GAUTHIER, Michel, La Prairie, Québec J5R 1E6 (CA); RAVET, Nathalie, Montréal, Québec H3T 1T9 (CA)
(74) Mandataire: Bertrand, Didier
(86) Numéro de dépôt international: PCT/CA1999/000087
(87) Numéro de publication internationale: WO 1999/040025

(56) Documents cités:
- EP-A- 0 834 892
- US-A- 5 538 812
- P.L. DHINGRA, ET AL.: "Chemistry of imidobis(sulphuryl fluoride): Part II - Behaviour of electrolytes in imidobis(sulphuryl fluoride)" JOURNAL OF THE INDIAN CHEMICAL SOCIETY, SECTION A: INORGANIC, PHYSICAL, THEORETICAL AND ANALYTICAL, vol. 24A, no. 6, juin 1985, pages 472-475, XP002104844 Calcutta, IN
- H.W. ROESKY, ET AL.: "Darstellung und Untersuchung von Fluorsulfurylverbindungen" CHEMISCHE BERICHTE, vol. 101, no. 1, 1968, pages 162-172, XP002104843 Weinheim, DE
- H.W. ROESKY, ET AL.: "Phosphor- und Schwefelhydrazine-Verbindungen" ZEITSCHRIFT FÜR NATURFORSCHUNG, TEIL B: CHEMIE, BIOCHEMIE, BIOPHYSIK, BIOLOGIE, vol. 26b, no. 12, 1971, pages 1232-1235, XP002104845 Tübingen, DE
- H.W. ROESKY, ET AL.: "Darstellung und Reaktionen des 1,2,3,5-dithiadiazoliumchlorids" CHEMISCHE BERICHTE, vol. 111, no. 8, 1978, pages 2960-2964, XP002104842 Weinheim, DE
- H.W. ROESKY, ET AL.: "Preparation and reactions of fluorosulphonyliminosulphuroxy difluoride" INORGANIC CHEMISTRY., vol. 8, no. 8, août 1969, pages 1733-1735, XP002104847 Washington, DC, US
- W. ISENBERG, ET AL.: "Tetraphenylarsonium bis(fluorosulphonyl)amide" ACTA CRYSTALLOGRAPHICA, vol. B38, no. 11, 15 octobre 1982, pages 2887-2889, XP002104846 Copenhague, DK

## Description

### DOMAINE DE L'INVENTION

La présente invention a pour objet des compositions ioniques ayant une conductivité ionique élevée, comprenant un sel dans lequel la charge anionique est délocalisée, et leurs utilisations, notamment comme électrolyte.

### ART ANTÉRIEUR

On connaît depuis longtemps les sels fondus à température ambiante, tel que le nitrate de triéthyle ammonium. Ce composé ne présente pas d'intérêt autre que fondamental du fait de la présence d'un proton labile sur le cation, limitant le domaine de stabilité rédox ou acido-basique de ce composé. On connaît aussi les composés de type méthyl-éthylimidazolium ou butyl-pyridinium, associés à l'ion complexe [Cl⁻, xAlCl3] dans lequel 1< x < 2. Ces composés, du fait de la présence de chlorure d'aluminium sont des acides de Lewis très puissants en plus d'être hygroscopiques et corrosifs car ils libèrent de l'acide chlorhydrique en présence d'humidité. Leur domaine de stabilité électrochimique est limité de plus par l'oxydation anodique des ions chlorure d'une part, et par la réduction des ions aluminium d'autre part.

L'utilisation d'anions réputés stables associés à des cations de type imidazolium ou pyridinium a été proposée, mais les points de fusion sont relativement élevés. Par exemple, l'hexafluorophosphate de 1-méthyl-3-éthylimidazolium fond à 60°C, et l'hexafluorophosphate de 1,2-diméthyl-3-propylimidazolium fond à 65°C. De plus, ces sels, bien que non hygroscopiques, sont néanmoins solubles dans l'eau et peuvent donc difficilement être préparés par échange ionique dans l'eau à moins d'utiliser des substituants alkyles plus longs, ce qui a pour effet de fortement diminuer la conductivité et d'augmenter la viscosité.

US 5,827,602 décrit des sels ayant un point de fusion relativement bas, et dont le critère de choix est un volume de l'anion supérieur à 100 Å³, permettant ainsi d'obtenir des sels à caractère hydrophobe et de conductivité élevée. Les anions les plus représentatifs sont le bis-trifluorométhanesulfonimidure, dont le volume tel que calculé par le programme Hyperchem® est de 144 Å³, ou le tris-trifluorométhanesulfonylméthylure, dont le volume est de 206 Å³.

### SOMMAIRE DE L'INVENTION

La présente invention concerne des composé ioniques de bas point de fusion, préférablement inférieur à la température ambiante, dont le cation est de type onium et possède au moins un hétéroatome tel que N, O, S ou P portant la charge positive et dont l'anion inclut, en totalité ou en partie, au moins un ion imidure du type (FX¹O)N⁻(OX²F) dans lequel X¹ et X² sont identiques ou différents et comprennent SO ou PF. Plus spécifiquement, le cation de type onium comprend un composé de formule : 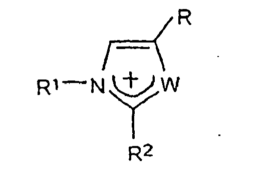 un composé de formule 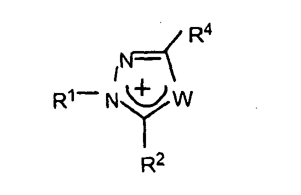 un composé de formule 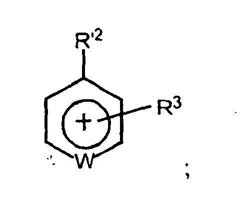 un composé de formule 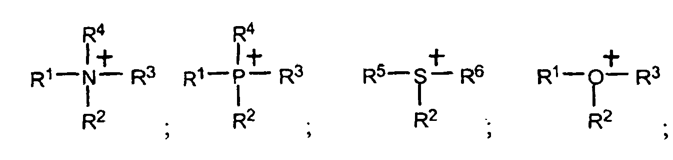 ou 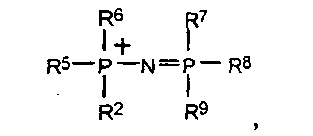 dans lesquels
W est O, S ou N, et dans lequel N est optionnellement substitué par R¹ lorsque la valence le permet;
- R¹, R³, R⁴ sont identiques ou différents et représentent
- H;
- les radicaux alkyles, alkényles, oxaalkyles, oxaalkényles, azaalkyles, azaalkényles, thiaalkyles, thiaalkényles, dialkylazo, lesdits radicaux pouvant être linéaires, ramifiés ou cycliques et comprenant de 1 à 18 atomes de carbone;
- les radicaux cycliques ou hétérocycliques aliphatiques de 4 à 26 atomes de carbone comprenant optionnellement au moins une chaîne latérale comprenant un ou plusieurs hétéroatomes;
- les groupes comprenant plusieurs noyaux aromatiques ou hétérocycliques, condensés ou non, contenant optionnellement un ou plusieurs atomes d'azote, d'oxygène, de soufre ou de phosphore,
   deux groupements R¹, R³ ou R⁴ pouvant.former un cycle ou un hétérocycle de 4 à 9. atomes un ou plusieurs groupements R¹, R³ ou R⁴ sur un même cation peuvent faire partie d'une chaîne polymère;
- R² et R⁵ à R⁹ sont identiques ou différents et représente R¹, R¹O-, (R¹)₂N-, R¹S-, R¹ étant tel que défini précédemment.

L'invention comprend en outre une composition électrolytique comprenant au moins un composé ionique tel que défini ci-haut en combinaison avec au moins un autre composant comprenant un sel métallique, un polymère polaire et/ou un co-solvant aprotique.

### DESCRIPTION DÉTAILLÉE DE L'INVENTION

Il a été trouvé que les sels de cations de type onium variés tels que définis ci-dessus, et préférablement les sels imidazolium, ammonium, sulfonium et phosphonium, associés aux anions de la famille représentée par la formule générale (FX¹O)N⁻(OX²F) telle que définie ci-dessus permettent d'obtenir des sels liquides à des températures égales ou inférieures à celles obtenues avec des ions plus volumineux. De plus, leur conductivité est, dans tous les cas, à température identique, supérieure à celle des composés décrits dans US 5,827,602. Ces sels liquides sont hydrophobes malgré la faible taille de l'anion, comprise entre 85 et 92 A³, et donc facilement préparés par échange ionique dans l'eau, et peuvent être manipulés sans précaution particulière. De façon inattendue, ces sels ont une stabilité en oxydation égale à celle des anions bis(trifuorométhanesulfonimidure) ou tris(trifuorométhanesulfonyl)méthylure, et supérieure à celle obtenue avec les anions de type tétrafluoroborate ou hexafluorophosphate.

Les composés de la présente invention peuvent, en plus de l'anion imidure précité, comprendre au moins un autre anion choisi parmi Cl⁻; Br⁻; I⁻; NO₃⁻; M(R¹⁰)₄⁻ A(R¹⁰)₆⁻; R¹¹O₂⁻, [R¹¹ONZ¹]⁻, [R¹¹YOCZ²Z³]⁻, le 4,5-dicyano-1,2,3-triazole, le 3,5-bis(R_{F})-1,2,4-triazole, le tricyanométhane, le pentacyanocyclopentadiène, le pentakis-(trifluorméthyl)cyclopentadiène, les dérivés de l'acide barbiturique et de l'acide de Meldrum et leurs produits de substitutions;
- M est B, Al, Ga ou Bi;
- A est P, As et Sb;
- R¹⁰ est un halogène;
- R¹¹ représentant H, F, un groupement alkyle, alkényle, aryle, arylalkyle, alkylaryle, arylalkényle, alkénylaryle, dialkylamino, alcoxy ou thioalcoxy, chacun ayant de 1 à 18 atomes de carbone et étant non substitué ou substitué par un ou plusieurs substituants oxa, thia, ou aza, et dans lesquels un ou plusieurs atomes d'hydrogène sont optionnellement remplacés par un halogène dans une proportion de 0 à 100%, et pouvant éventuellement faire partie d'une chaîne polymère;
- Y représentant C, SO, S=NCN, S=C(CN)₂, POR¹¹, P(NCN)R¹¹, P(C(CN)₂R¹¹, un groupement alkyle, alkényle, aryle, arylalkyle, alkylaryle, arylalkényle, alkénylaryle possédant de 1 à 18 atomes de carbone et optionnellement substitué par un ou plusieurs substituants oxa, thia ou aza; un groupement dialkylamino N(R¹⁰)₂;
Z¹ à Z³ représentent indépendamment R¹¹, R¹¹YO ou CN, ce groupement pouvant optionnellement faire partie d'une chaîne polymère.

Un autre avantage des composés ioniques de l'invention est le moindre coût des anions de départ, leur préparation ne faisant pas appel à la chimie des perfluoroalkyles tel que CF₃ ou C₄F₉ par exemple, les atomes de fluor présents dans les composés de l'invention étant dérivés de produit de la chimie inorganique, donc facilement accessibles. Cet aspect économique est particulièrement important car les sels fondus sont constitués de 40 à 75 % en poids de l'espèce anionique, le reste étant l'espèce cationique. De plus, la densité de ces liquides est proche de 1.5 comparé à environ 1 pour les solutions organiques, ce qui requiert des quantités d'autant plus importantes de sels pour toutes les applications où un volume ou une épaisseur donnée sont requises, telles que les film d'électrolytes, réacteurs chimiques etc.

Il est particulièrement important que ces sels fondus ont la faculté de dissoudre d'autres sels, en particulier des sels métalliques, notamment les sels de lithium pour donner des solutions très conductrices. D'une manière similaire les sels fondus, ou leurs mélanges avec d'autres sels métalliques, sont d'excellents solvants ou plastifiants d'un grand nombre de polymères, en particulier ceux portant des fonctions polaires ou ioniques. Aussi bien les composés liquides que les polymères plastifiés par les mélanges ioniques se comportant comme des électrolytes solides sont applicables en électrochimie aux générateurs de type primaire ou secondaires, aux supercapacités, aux systèmes électrochromes, aux revêtements antistatiques, ou encore aux diodes électroluminescentes. La non-volatilité des sels fondus de l'invention, leur stabilité thermique et électrochimiques, et leur conductivité accrue sont des paramètres importants pour la réalisation de systèmes fonctionnant à basse température et ne présentant pas les risques d'inflammabilité habituels liés à l'emploi de solvants organiques usuels.

Les sels fondus de l'invention sont des milieux polaires de faibles volatilité, et par cette propriété, sont capables de servir de solvant permettant d'effectuer un grand nombre de réactions de la chimie organique, telles les substitutions nucléophiles et électrophiles, ou encore les polymérisations anioniques, cationiques ou radicalaires. Il est de plus possible de dissoudre dans ce milieu des catalyseurs, en particulier des sels de métaux de transition ou des terres rares éventuellement cordonnés par des ligands, permettant d'exhalter les propriétés catalytiques. Des exemples de ces catalyseurs incluent les bipyridines, les porphyrines, les phosphines, les arsines. Les organométalliques tels que les métallocènes sont aussi inclus comme solutés pouvant présenter des propriétés catalytiques.

La non-volatilité des sels fondus de l'invention, leur stabilité thermique ainsi que leur non miscibilité avec les solvants non polaires comme les hydrocarbures, de même que leur caractère hydrophobe, sont particulièrement avantageux pour séparer les produits de réactions chimiques. Il est de même possible de travailler en systèmes diphasiques, le sel fondu contenant le catalyseur et les substrats réactifs étant en solution dans un hydrocarbure ou éther aliphatique non miscible. Après la réaction, une simple décantation permet de séparer la phase organique contenant le produit de la réaction et le sel fondu qui est purifié par lavage avec un non solvant tel que l'eau ou un hydrocarbure, et séché par simple mise sous vide.

Par ailleurs, les cations ammonium, phosphonium et sulfonium peuvent présenter une isomérie optique, et les sels fondus les contenant sont des solvants chiraux susceptibles de favoriser la formation d'excès énantiomériques dans les réactions effectuées dans ces milieux: Les cations préférés comprennent les composés de formule: 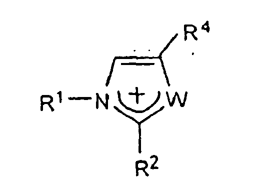 qui incluent les dérivés imidazolium, triazolium, thiazolium, et oxazolium; les composés de formule 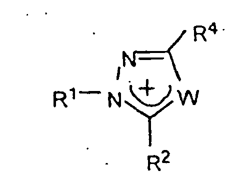 qui incluent les dérivés trizolium, oxadiazolium, et thadiazolium; les composés de formule 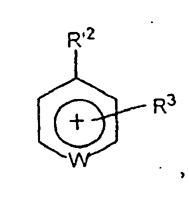 préférentiellement les dérivés 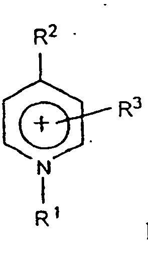 pyridinium; les composés de formule 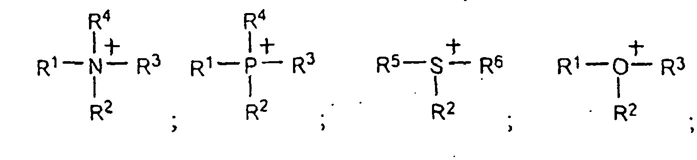 ou 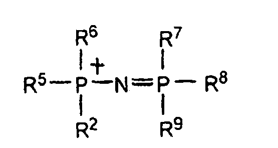 dans lesquels
W est O, S ou N, et dans lequel N est optionnellement substitué par R¹ lorsque la valence le permet;
R¹, R³, R⁴ sont identiques ou différents et représentent
- H;
- les radicaux alkyles, alkényles, oxaalkyles, oxaalkényles, azaalkyles, azaalkényles, thiaalkyles, thiaalkényles, dialkylazo, lesdits radicaux pouvant être linéaires, ramifiés ou cycliques et comprenant de 1 à 18 atomes de carbone;
- les radicaux cycliques ou hétérocycliques aliphatiques de 4 à 26 atomes de carbone comprenant optionnellement au moins une chaîne latérale comprenant un ou plusieurs hétéroatomes tels que l'azote, l'oxygène ou le soufre;
- les aryles, arylalkyles, alkylaryles et alkénylaryles de 5 à 26 atomes de carbone comprenant optionnellement un ou plusieurs hétéroatomes dans le noyau aromatique;
- les groupes comprenant plusieurs noyaux aromatiques ou hétérocycliques, condensés ou non, contenant optionnellement un ou plusieurs atomes d'azote, d'oxygène, de soufre ou de phosphore,
   deux groupements R¹, R³ ou R⁴ pouvant former un cycle ou un hétérocycle de 4 à 9 atomes un ou plusieurs groupements R¹, R³ ou R⁴ sur un même cation peuvent faire partie d'une chaîne polymère; et
- R² et R⁵ à R⁹ sont identiques ou différents et représente R¹, R¹O-, (R¹)₂N-, R¹S-, R¹ étant tel que défini précédemment.

Les groupements R¹, R³ et R⁴ peuvent porter des groupements actifs en polymérisation tel des doubles liaisons ou des époxydes, ou des fonctions réactives dans les polycondensations, telles que OH, NH₂ et COOH. Dans le cas où les cations portent des doubles liaisons, ils peuvent être homopolymérisés ou copolymérisés, par exemple avec du fluorure de vinylidène, un acrylate, une maléimide, de l'acrylonitrile, un vinyléther, un styrène, etc. Les groupements époxydes peuvent être polycondensés ou copolymérisés avec d'autres époxydes. Ces polycations sont particulièrement utiles seuls ou en mélange avec un solvant, y compris un sel fondu de la présente invention et/ou un ou plusieurs sels sel de lithium ou un mélange de sels de lithium et potassium comme électrolyte dans les batteries au lithium à anode de lithium ou utilisant une cathode insérant le lithium à bas potentiel comme les spinelles de titane ou des matériaux carbonés.

L'invention envisage en outre une composition électrolytique comprenant au moins un composé ionique comprenant au moins un anion et au moins un cation tels que définis précédemment en combinaison avec au moins un autre composant comprenant un sel métallique, un polymère polaire et/ou un co-solvant aprotique. Le cation préférentiel du sel métallique comprend le proton, le cation d'un métal alcalin, d'un métal alcalino-terreux, d'un métal de transition ou d'une terre rare, le lithium étant tout particulièrement préféré.

Le polymère polaire préféré comprend des unités monomères dérivées de l'oxyde d'éthylène, l'oxyde de propylène, l'épichlorohydrine, l'épifluorohydrine, le trifluoroépoxypropane, l'acrylonitrile, le méthacrylonitrile, les esters et amides de l'acide acrylique et méthacrylique, le fluorure de vinylidène, la N-méthylpyrrolidone et les polyélectrolytes de type polycation ou polyanion. Finalement, des exemples de co-solvant aprotique préférentiels sont les éthers di-alkyliques de l'éthylène glycol, du diéthylène glycol, du triéthylène glycol, des polyéthylène glycols de masse comprise entre 400 et 2000; les esters, en particulier ceux de l'acide carbonique, linéaires ou cyclique tels le diméthylcarbonate, le méthyl-éthylcarbonate, le diéthylcarbonate, le carbonate d'éthylène, le carbonate de propylène ; les esters comme la γ-butyrolactone, les nitriles comme le glutaronitrile, le 1,2,6-tricyanohexane , les amides comme le diméthylformamide, la N-méthylpyrrolidinone, les sulfamides et sulfonamides ainsi que les mélanges des composés précités.

Lorsque la présente composition électrolytique comprend plus d'un polymère, au moins un de ces derniers peut être réticulé.

Un générateur électrochimique comprenant une composition électrolytique de l'invention contient préférentiellement une électrode négative contenant soit du lithium métallique ou un de ses alliages, soit un composé d'insertion du carbone, en particulier du coke de pétrole ou du graphite, soit un oxyde à bas potentiel d'insertion tel que les spinelles de titane Li_{4-x+3y}Ti₅₋ₓO₁₂ (0≤ x, y≤ 1), soit un nitrure double d'un métal de transition et de lithium comme Li₃₋ₓCo_{z}N (0≤ z≤ 1) ou ayant la structure de type antifluorite comme Li₃FeN₂ ou Li₇MnN₄, ou leurs mélanges. L'électrode positive du générateur contient préférentiellement soit de l'oxyde de vanadium VOₓ (2≤ x ≤ 2,5), soit de l'oxyde mixte de lithium et de vanadium LIV₃O₈, soit un oxyde double de cobalt et de lithium optionnellement partiellement substitué de formule générale Li_{1-α}Co_{1-x+y}NiₓAl_{y}(0≤ x + y≤ 1 ; 0 ≤ y≤ 0,3 ; 0 ≤ α≤ 1), soit un spinelle de manganèse optionnellement partiellement substitué de formule générale Li_{1-α}Mn_{2-z}M_{z} (0≤ z ≤ 1) où M = Li, Mg, Al, Cr, Ni, Co, Cu, Ni, Fe, soit un phosphate double de structure olivine ou Nasicon tels que Li_{1-α}Fe₁₋ₓMnₓPO₄, Li_{1-x+2α}Fe₂P₁₋ₓSiₓO₄ (0≤ x, (α≤ 1), soit un sel de l'acide rhodizonique, soit un polydisulfure dérivé de l'oxydation du dimercaptoéthane, du 2,5-dimercapto-1,3,4-thiadiazole, 2,5-dimercapto-1,3,4-oxadiazole, du 1,2-dimercaptocyclobutène-3,4-dione, ou leurs mélanges.

De façon avantageuse, au moins une des électrodes du générateur est mélangée à la composition électrolytique pour former une électrode composite.

La composition électrolytique de l'invention peut en outre être utilisée comme électrolyte dans un système de stockage de l'énergie électrique de type supercapacité, contenant optionnellement dans une électrode du carbone de grande surface spécifique, ou un polymère conjugué. De façon avantageuse, le polymère conjugué contient 3 degrés d'oxydation, et se retrouve dans les 2 électrodes. Un exemple d'un tel polymère est un dérivé du phényl-3-thiophène.

Finalement, la composition électrolytique de l'invention peut être utilisée comme électrolyte dans un système de modulation de la lumière de type électrochrome comprenant au moins un matériau électrochrome. Dans un tel système, le matériau électrochrome est avantageusement déposé sur une couche d'un semi-conducteur transparent dans le visible, préférentiellement un dérivé de l'oxyde d'étain ou de l'oxyde d'indium, sur un substrat de verre ou d'un polymère. Des exemples de matériaux électrochromes préférentiels incluent l'oxyde de molybdène, de tungstène, de titane, de vanadium, de niobium, de cérium, d'étain, ainsi que leurs mélanges. Le matériau électrochrome peut être optionnellement dissous dans l'électrolyte.

Les exemples suivants sont fournis afin d'illustrer des mises en oeuvre préférentielle de l'invention, et ne doivent pas être considéré comme en limitant sa portée.

### Exemple 1

15 g de chlorure de 1-méthyl-3-éthyl imidazolium EMICl (C₆H₁₁N₂Cl) sont dissous dans 100 ml d'eau auquel sont ajoutés sous agitation 23 g de bis-fluorosulfonimidure de potassium (KFSI) K[(FSO₂)₂N]. Une séparation en deux phases liquide se produit immédiatement. Le sel fondu bis-fluorosulfonimidure de 1-méthyl-3-éthyl imidazolium (EMIFSI) est extrait par le dichlorométhane et séché par du sulfate de magnésium anhydre. La suspension est filtrée et le solvant évaporé. Le sel est séché sous vide à 80°C correspond à la formule développée :

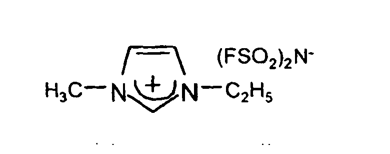

Ce composé ionique examiné par DSC présente un point de fusion de -15°C. La perte de poids mesurée par analyse thermique différentielle (ATD) sous argon est inférieure à 1% jusqu'à 350°C. La conductivité un fonction de la température est donné dans le Tableau 1 ci-dessous:

**TABLEAU 1**

| | | | | | | | | |
|---|---|---|---|---|---|---|---|---|
| Température (°C) | -10 | 0 | 10 | 20 | 30 | 40 | 50 | 60 |
| Conductivité (mScm⁻¹) | 5.6 | 8.3 | 11 | 15 | 20 | 25 | 31 | 37 |
| σ (FSI) / σ TFSI (%) | 2.41 | 2.21 | 2.01 | 1.86 | 1.80 | 1.70 | 1.67 | 1.64 |

La conductivité est supérieure à celle obtenue avec le sel bis-trifluorométhanesulfonimidure de 1-méthyl-3-éthyl imidazolium (EMITFSI). Le rapport entre les valeurs de conductivité σ entre le sel de l'invention, i.e, fluorosulfonimidure de 1-méthyl-3-éthyl imidazolium (noté FSI dans le tableau) et bis-trifluorométhanesulfonimidure de 1-méthyl-3-éthyl imidazolium (noté TFSI dans le tableau) est donné dans la dernière ligne du tableau précédent. Ces chiffes montrent la très nette amélioration des performances de conductivité par rapport à l'art antérieur.

Le domaine de stabilité électrochimique mesurée par voltammétrie cyclique sur électrode de nickel pour les potentiels cathodiques et de carbone vitreux pour les potentiels anodiques est de 5.2 Volts (0 ⇒ 5.2 V vs. Li⁺/Li^{°}).

### Exemple 2

Le bis-difluorophosphonylamidure de lithium Li[(POF₂)₂N] est préparé selon la méthode de Fluck et Beuerle dans *Z. Anorg. Allg. Chem.,* 1975, 412, 65, par réaction du dérivé lithié de l'hexaméthyldisilazane sur l'oxyfluorure de phosphore selon la réaction suivante:

2 POF₃ + Li[Si(CH₃)₃]₂N ⇒ 2 FSi(CH₃)₃ +Li[(POF₂)₂N]

Le sel ionique fondu bis-difluorophosphonylimidure de 1-méthyl-3-éthyl imidazolium est préparé par échange ionique dans l'eau de selon l'exemple 1 entre 10 g de EMICl et 13 g Li[(POF₂)₂N] et extraction au dichlorométhane. Le sel fondu a des propriétés physico-chimiques similaires à celles du sel de fluorosulfonyle de l'exemple 1.

### Exemple 3

Différents sels d'imidazolium de formule générale: 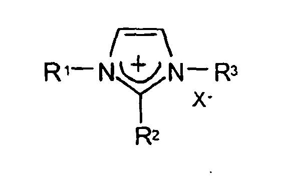 ont été préparés avec les anions [(FSO₂)₂N]⁻ et [(POF₂)₂N]⁻, et sont illustrés dans le Tableau 2 ci-dessous. Ceux avec un indice "+" sont des sels liquides à température ambiante.

### C7H15 ou C7H13?

**TABLEAU 2**

| R³ = | CH₃ | C₂H₅ | C₃H₇ | C₄H₉ | C₅H₁₁ | C₇H₁₅ | C₈H₁₇ |
|---|---|---|---|---|---|---|---|
| R¹= CH_{3,} R² = H | | + | + | + | + | + | + |
| R¹= CH_{3,} R² = CH₃ | | | + | + | + | + | + |
| R¹= C₂H_{5,} R² = H | + | + | + | + | + | + | + |
| R¹= CH_{3,} R² = C₃H₃ | + | + | + | + | + | + | + |

### Exemple 4

10 g de bromure de triéthylhéxyl ammonium commercial (C₁₂H₂₈NBr) sont dissous dans 150 ml d'eau auxquels sont ajoutés sous agitation 8.5 g de bisfluorosulfonimidure de potassium [K(FSO₂)₂N]. Le sel fondu bis-fluorosulfonimidure de triéthylhéxyl ammonium est séparé par centrifugation et lavé par trois aliquotes de 50 ml d'eau puis extrait par 30 ml de dichlorométhane et séché par du sulfate de magnésium anhydre. La suspension est filtrée et le solvant évaporé, laissant un liquide visqueux. La conductivité à 25°C est supérieure à 5 x 10⁻⁴ Scm⁻¹ à 25°C.

### Exemple 5

10 g de diméthyléthylamine commerciale et 11 ml de bromo-1-propane sont mis au reflux dans 40 ml d'acétonitrile pendant 48 heures. Le solvant est.ensuite évaporé et le résidu solide est lavé à l'éther. À 12 g du sel (CH₃)₂(C₂H₅)(C₃H₇)NBr dissous dans 75 ml d'eau sont ajoutés 13 g de bis-fluorosulfonimidure de potassium [K(FSO₂)₂N]. Le sel fondu est extrait comme précédemment et se présente sous forme d'un liquide peu visqueux. Sa conductivité en fonction de différentes températures est donnée dans le Tableau 3 suivant.

**Tableau 3**

| Température (°C) | | 20 | 30 | 40 | 50 |
|---|---|---|---|---|---|
| conductivité (mScm⁻¹) | FSI | 4,95 | 7,02 | 9,4 | 12,3 |
| | TFSI | 1,81 | 2,92 | 4,3 | 6,1 |

A titre de comparaison, la valeur de la conductivité du sel bis-trifluorométhanesulfonimidure de diméthyléthylpropyl-ammonium est donnée (Tableau 3, ligne 3). La conductivité du composé selon l'invention apparaît de 2,5 à 2 fois plus élevée que celle du sel équivalent faisant intervenir un anion plus volumineux.

### Exemple 6

La N-méthyl-N-éthyl-aniline est quatemarisée par le bromopropane au reflux dans l'acétonitrile pendant 48 heures. Le sel est obtenu par évaporation du solvant et purifié par lavage du résidu solide à l'éther. 5 g du sel obtenu sont dissous dans 25 ml d'eau, et 4.6 g de bis-fluorosulfonimidure de potassium (K(FSO₂)₂N) y sont ajoutés. Le sel fondu bis-fluorosulfonimidure de méthyléthylpropylphényl ammonium est extrait par 15 ml de dichlorométhane, lavé par trois aliquotes de 50 ml d'eau et séché par du sulfate de magnésium anhydre. Ce sel existe sous deux isomères optiques qui peuvent être séparés sur une colonne chirale ou par précipitation du sel de camphre-sulfonate à partir du bromure avant échange par l'imidure. Ce sel peut servir de milieu réactionnel chiral.

### Exemple 7

Le bromure de N-butylpyridinium est préparé par action du bromobutane sur la pyridine en l'absence de solvant à 45°C. À 5 g de sel dissous dans 35 ml d'eau sont ajoutés 4,6 g de bis-diflurophosphonylimidure de lithium Li[(POF₂)₂N]. Le sel liquide est traité d'une manière similaire à celle des sels fondus obtenus dans les exemples précédents et est finalement séché sous vide à 60°C. Le sel fondu bis-fluorosulfonimidure de N-propyl pyridinium est préparé d'une manière similaire à partir du sel de potassium correspondant.

### Exemple 8

Le sulfure d'éthylméthyle commercial (Aldrich, Milwaukee USA) est quaternarisé par le sulfate de propyle (TCI, Japon). Le propylsulfate de diéthylméthylpropylsulfonium est traité en solution aqueuse par 1 équivalent de bis-fluorosulfonimidure de potassium. Le sel fondu liquide est extrait comme précédemment. D'une manière similaire à celle employée pour l'exemple 4, ce sel peut être dédoublé en deux isomères optiquement actifs et servir à induire un excès énantiomérique pour les réactions effectuées lorsque le sel est utilisé comme solvant.

### Exemple 9

15 g de chlorhydrate de 4-chloropyridine commercial sont dissous dans 100 ml d'eau auxquels sont ajoutés 8.5 g de bicarbonate de sodium. La 4-chloropyridine est extraite par l'éther et séchée par le sulfate de magnésium, et le solvant est évaporé. 10 g de 4-chloropyridine dans 60 ml d'acétonitrile sont quatemarisés par 15.6 g de trifluorométhanesulfonate d'éthyle et sont ajoutés 11.6 g de triméthylsilyléthylméthylamine C₂H₅(CH₃)NSi(CH₃)₃. Le mélange réactionnel est mis au reflux pendant une heure puis refroidi. Le solvant est évaporé et le résidu solide est repris par l'eau. À cette solution sont ajoutés 19.5 g de bis-fluorosulfonimidure de potassium. Le sel liquide qui se décante est extrait au dichlorométhane. Ce sel a pour structure:

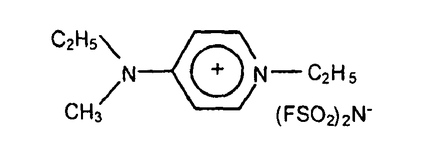

### Exemple 10

La fluorosulfonamide FSO₂NH₂ est préparée par action de l'anhydride fluorosulfonique (15 g) sur la carbamate d'ammonium (12 g) en suspension dans le dichlorométhane selon la réaction suivante:

(FSO₂)₂O + 1,5H₂NCO₂(NH₄) ⇒ FSO₃(NH₄) + 1,5CO₂ + FSO₂NH(NH₄)

Le mélange réactionnel est filtré. L'amide FSO₂NH₂ est libérée par l'acide chlohrydrique dilué et extait à l'éther. Le dérivé triméthylsilylé du dérivé sodique de la fluorosulfonamide est préparé par la méthode de Foropoulous et al. dans *Inorganic Chem.,* 1984, 23, 3720. Dans un réacteur Parr® sont mis 80 ml d'acétonitrile anhydre et 10 g du dérivé de la fluorosulfonamide. Le réacteur est fermé et purgé sous azote et sont admis 5.38 g de fluorure de phosphoryle POF₃ en maintenant la température à 45°C. La pression tombe après une heure et le réacteur est refroidi et ouvert. Le sel de sodium de l'imide mixte a été obtenu selon la réaction

NaNSi(CH₃)₃SO₂F+ POF₃ ⇒ Si(CH₃)₃ + Na[(F₂PO)(FSO₂)N]

Le sel est recueilli par évaporation du solvant et recristallisé dans un mélange toluèné-acétonitrile: Ce sel donne des dérivés ioniques liquides avec les imidazolium de l'exemple 3 et présente un domaine de liquides plus large que celui des sels de bis-trifluorométhanesulfonimidure et une conductivité supérieure de 10 à 25%.

### Exemple 11

25 ml d'un solution commerciale de chlorure de diallyldiméthyl-ammonium à 65% dans l'eau sont dilués par 100 ml d'eau et sont ajoutés sous agitation 22 g de bis-fluorosulfonimidure de potassium. Le précipité liquide est extrait par le dichlorométhane et séché par le sulfate de magnésium. Ce sel fondu a pour formule développée :

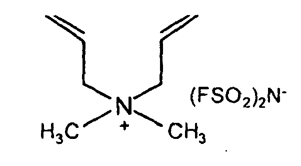

Il se comporte comme un monomère actif en polymérisation radicalaire pour former par cyclopolymérisation des motifs bis(3,5-méthylène-) diméthylpyrrolidinium. Ce composé donne, outre des homopolymères, des copolymères avec le stryrène, l'anhydride maléique, les N-maléimides, le fluorure de vinylidène, l'acrylonitrile, le méthacrylonitrile, le méthacrylate de méthyle, avec les acrylates ou méthacrylates de ω-methoxyoligo ethylène glycols de masse comprise entre 200 et 2000 daltons, éventuellement réticulés par un diacrylate ou méthacrylate de α, ω-oligo ethylène glycol.

### Exemple 12

5 g de pentachlorure de phosphore sont dissous dans 50 ml de dichlorométhane dans un ballon muni d'une ampoule à brome et d'une entrée d'argon sec. Le mélange est refroidi par de la glace carbonique à -78°C et on ajoute goutte à goutte 20 ml de méthyléthylamine dans 30 ml d'acétonitrile anhydre par l'ampoule à brome. Le mélange réactionnel est maintenu sous agitation pendant 1 heure en le laissant revenir à température ordinaire. Le solvant est ensuite évaporé et le résidu est repris dans 75 ml d'eau et filtré sur Celite®. À cette solution sont ajoutés 5.5 de bis-fluorosulfonimidure de potassium. Le milieu réactionnel se sépare en deux phases liquides. Le sel fondu de tétrakis(éthylméthylamino)phosphonium {P[N(CH₃)(C₂H₅)]₄}⁺[(FSO₂)₂N]⁻ est un liquide huileux à température ordinaire. Ce sel fondu est particulièrement stable vis-à-vis des agents réducteurs ou nucléophiles, et ce, même à des températures élevées.

### Exemple 13

20 g d'une solution aqueuse commerciale à 25% de poly(chlorure de diallyldiméthylammonium) de haute masse moléculaire (M_{w} environ 2 x 10⁵) sont dilués dans 100 ml d'eau. Sous agitation magnétique sont ajoutés 6.7 g de bis-fluorosulfonimidure de potassium dans 100 ml d'eau. Le précipité de poly(bis-fluorosulfonimidure de diallyldiméthyl-ammonium) 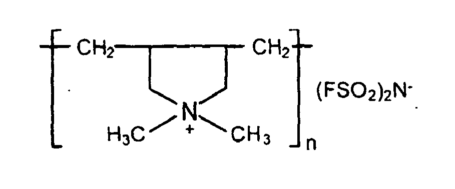 est ensuite filtré et lavé abondamment à l'eau distillée, puis séché sous vide.

### Exemple 14

Un électrolyte liquide est obtenu par dissolution du bis-trifluorométhanesulfonylimidure de lithium (LiTFSI) en concentration 1 molaire dans le sel fondu préparé selon l'exemple 1. La conductivité de ce mélange est de 9 x 10⁻³ Scm⁻¹ à 25°C, et reste supérieure à 2 x 10⁻³ Scm⁻¹ à 0°C. Par voltammétrie cyclique, le domaine anodique est trouvé supérieur à 5 V/Li^{°}/Li⁺.

### Exemple 15

Un électrolyte solide est obtenu par plastification du polyélectrolyte de l'exemple 13 par la solution de sel de lithium dans le sel d'imidazolium de l'exemple 14. Pour la mise en forme de cet électrolyte, les 3 composants (polyélectrolyte-FSI, imidazolium-FSI, LiTFSI sont pesés de manière à respecter les proportions suivantes: polyélectrolyte (40 % en poids; LiTFSI, 1 M dans le sel d'imidazolium (60 % en poids). Les trois composants sont dissous dans un solvant polaire volatile, tel que l'acétonitrile, la quantité de solvant étant ajustée de manière à permettre l'épandage en film mince de la solution pour donner après séchage une épaisseur de 35 µm sur un support de polypropylène.

Le film ainsi obtenu est séché par un courant d'air sec, puis sous vide primaire à 100°C pendant deux heures. Toutes les manipulations successives de ce film sont faites en boîte à gants (< 1 ppm O₂ et H₂O). La conductivité de cet électrolyte est égale à 10⁻³ Scm⁻¹ à 20°C; 4×10⁻⁴ Scm⁻¹ à 0°C; et 3×10⁻³ Scm⁻¹ à 60°C. Des électrolytes de conductivité supérieure peuvent être obtenus en augmentant la fraction de plastifiant (> 60%), i.e., la solution de LiTFSI dans le sel fondu de l'exemple 1. D'une manière similaire, des modules d'élasticité supérieurs sont obtenus pour des fractions de plastifiant inférieures à < 50% avec un diminution de la conductivité.

### Exemple 16

Un électrolyte polymère de conductivité élevée est obtenu par plastification à raison de 40% en poids par le composé ionique de l'exemple 2 d'un complexe polyéther-sel métallique. Le complexe est à base de bis-trifluorométhanesulfonimidure de lithium et de poly(oxyde d'éthylène) de masse moléculaire 5 x 10⁶, tel que le rapport des oxygènes du polymère au nombre d'ions lithium soit égal à 20 (O : Li = 20:1). L'électrolyte peut être préparé directement par co-dissolution des composants pesés selon les proportions stoechiométriques dans un solvant tel que l'acétonitrile et évaporation suivie d'un séchage sous vide à 80°C.

Dans un variante, l'homopolymère d'oxyde d'éthylène peut être remplacé par un copolymère d'oxyde d'éthylène et d'allylglycidyléther (5% molaire) auquel sont ajoutés 1% en poids d'Irgacure 651®. La solution dans l'acétonitrile est épandue sur un support de polypropylène à l'aide d'un gabarit pour former un film de 20 microns d'épaisseur après séchage. Sous balayage d'argon, le film est soumis au rayonnement UV produit par une lampe de type Hanovia® ayant son maximum d'émission à 254 nm. L'éclairement correspond à une énergie de 130 mWcm⁻². Le polymère se réticule par un processus radicalaire par les segments insaturés et présente alors d'excellentes propriétés mécaniques de type élastomère. Des mélanges ternaires sel fondu / sel de lithium / polymère peuvent être obtenus d'une manière similaire avec comme matériau macromoléculaire soit l'acrylonitrile; soit le fluorure de polyvinylidène et ses copolymères avec l'hexafluoropropène, en particulier ceux solubles dans l'acétone et permettant une mise en oeuvre aisée; ou encore soit le polyméthacrylate de méthyle.

### Exemple 17

Un électrolyte polymère est préparé par polymérisation *in situ* d'un mélange du monomère de l'exemple 11 (25% en poids), du bis-trifluorométhanesulfonimidure de lithium (24%) et du sel fondu de l'exemple 1 (45%), et 1% de l'amorceur radicalaire 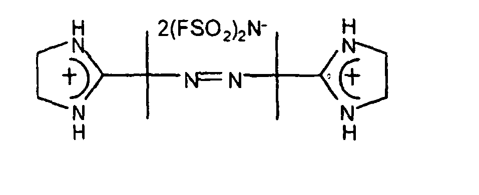 Cet amorceur est obtenu par échange dans l'eau à partir du chlorure commercial (Wako, Japon) et de K(FSO₂)₂N). Le mélange liquide est épandu à l'aide d'un gabarit sous forme de film de 30 microns d'épaisseur sur un support de polypropylène, et polymérisé dans un four tunnel sous atmosphère d'azote à 80°C pendant 1 heure. L'électrolyte ainsi obtenu est un élastomère possédant un domaine de stabilité anodique supérieur à 5V et une conductivité supérieure à 3 x 10⁻⁴ Scm⁻¹ à 25°C.

### Exemple 18

Un générateur électrochimique secondaire est fabriqué comprenant comme matériau actif de l'électrode positive de l'oxyde double cobalt et de lithium, LiCoO₂, et comme matériau actif de l'électrode négative le spinelle de titane et de lithium Li₄Ti₅O₁₂. L'électrolyte est préparé selon l'exemple 14 sous forme de film de 25 microns. Chaque électrode du type composite est préparée par épandage d'une suspension du matériau actif, de noir de carbone (Ketjenblack®) dans une solution dans le cyclohexane d'un copolymère d'éthylène-copropylène-diène (Aldrich, USA). La composition finale correspond à 90% en volume du matériau actif, 5% v/v de noir de carbone et 5% de copolymère. Après épandage sur collecteurs de courant en aluminium de 8 microns d'épaisseur, l'électrode négative contient 16.4 mg de matériau actif par cm² soit 2.9 mAhcm⁻², et l'électrode positive 16.5 mg de matériau actif par cm², soit 2.7 mAhcm⁻². Les électrodes et leur collecteur de courant sont découpés en carrés de 4 cm² et sont placées de part et d'autre d'une membrane de polyéthylène microporeux (Celgard®) imbibée de l'électrolyte liquide préparé selon l'exemple 14. La batterie ainsi assemblée est caractérisée par voltammétrie lente à l'aide d'un appareil de type MacPile® (Claix France). 92% de la capacité de l'électrode positive sont obtenus dans le domaine de voltage 2 - 2.8 V à une vitesse de balayage de 10 mV.mn⁻¹. La densité d'énergie dans cette configuration est de 85 Wh.kg⁻¹.

### Exemple 19

Un générateur électrochimique secondaire est fabriqué comprenant comme matériau actif de l'électrode positive le phosphate double de manganèse dopé au fer et de lithium, LiMn_{0,9}Fe_{0,1}PO₄, et comme matériau actif de l'électrode négative le spinelle de titane et de lithium Li₄Ti₅O₁₂. L'électrolyte est préparé selon l'exemple 15 sous forme de film de 25 microns. Chaque électrode du type composite est préparée par épandage d'une suspension de 45% en volume du matériau actif, 5% v/v de noir de carbone (Ketjenblack®) et d'une solution dans l'acétonitrile des composants de l'électrolyte selon l'exemple 12 (50% v/v). Les collecteurs de courant sont en aluminium de 8 microns d'épaisseur. Le collecteur de l'électrode positive est recouvert d'un enduit protecteur de graphite (Acheson, USA). Après épandage, l'électrode négative a une charge de 12 mg de matériau actif par cm² soit 2.2 mAhcm⁻², et l'électrode positive 14 mg de matériau actif par cm² soit 2.4 mAhcm⁻². Les électrodes et leur collecteurs de courant sont découpés en carrés de 4 cm² et sont placées de part et d'autre de l'électrolyte et l'assemblage est laminé à 80°C pour assurer un bon contact aux interfaces. La batterie ainsi assemblée est caractérisée par voltammétrie lente. 82% de la capacité de l'électrode positive sont obtenus dans le domaine de voltage 2.6 - 3.2 V à une vitesse de balayage de 10 mV.mn⁻¹. La densité d'énergie dans cette configuration est proche de 100 Wh.kg⁻¹.

### Exemple 20

Une supercapacité est élaborée à partir d'électrodes de fibres de carbone activé de 900 m²g⁻¹ (Spectracarb®). Deux carrés de 4 cm² sont découpés dans le tissus de carbone et sont imbibées sous vide par l'électrolyte préparé selon l'exemple 1. Les deux électrodes symétriques sont séparées par une membrane de polyéthylène poreux (Celgard®) imbibée sous vide du même liquide ionique. Les deux collecteurs de courant sont en aluminium de 10 µm recouvert par pulvérisation cathodique d'une couche protectrice de 5000 Å de molybdène. La capacité volumique pour une tension maximale de charge de 2.8 V est de 12 Fcm⁻³, soit 3 Wh.L⁻¹ au seuil de coupure de tension de 1.2 V.

### Exemple 21

Le sel d'imidazolium de l'exemple 1 est utilisé comme solvant du bis-trifluorométhanesulfonimidure d'yttrium en concentration de 0.1M. Ce liquide est utilisé comme catalyseur de la réaction de Diels-Alder du cyclopentadiène sur l'acrylate de méthyle. Les réactifs sont mélangés en quantité stoechiométrique, et le liquide ionique est ajouté à raison de 30% v/v. Sous agitation, la réaction est complétée en 1 heure à 25°C. Les produits de la réaction sont extraits avec de l'hexane, qui est non miscible avec le composé ionique. Le rapport endo/exo est de 9:1. Le catalyseur traité à 100°C sous vide peut être réutilisé sans perte de son activité.

### Exemple 22

Le sel fondu préparé selon l'exemple 12 est utilisé comme solvant pour des réactions de substitution nucléophile. 10 g de ce sel et 3 g de cyanure de potassium sont mis dans un tube de verre dans un four, et la température est portée à 250°C. 4 g de chlorure de benzyle sont chauffés à 60°C pendant 2 heures. La rendement de conversion du chlorure de benzyle en cyanure de benzyle est de 85%. Le sel fondu peut être aisément recyclé par lessivage à l'eau et évaporation.

Bien que la présente invention ait été décrite à l'aide de mises en oeuvre spécifiques, il est entendu que plusieurs variations et modifications peuvent se greffer aux dites mises en oeuvre, et la présente demande vise à couvrir de telles modifications, usages ou adaptations de la présente invention suivant, en général, les principes de l'invention et incluant toute variation de la présente description qui deviendra connue ou conventionnelle dans le champ d'activité dans lequel se retrouve la présente invention, et qui peut s'appliquer aux éléments essentiels mentionnés ci-dessus, en accord avec la portée de la revendication unique qui suit.

## Revendications

1. Solvant électrolytique de bas point de fusion dont le cation est de type onium possédant au moins un hétéroatome tel que N, O, S ou P portant la charge positive et dont l'anion inclut, en totalité ou en partie, au moins un ion imidure du type (FX¹O)N⁻(OX²F) où X¹ et X² sont identiques ou différents et comprennent SO ou PF.

## Claims

1. Low-melting electrolytic solvent whose cation is of the onium type having at least one heteroatom, such as N, O, S or P, carrying the positive charge, and whose anion includes, in whole or in part, at least one imide ion of the type (FX¹O)N⁻(OX²F), in which X¹ and X² are identical or different and comprise SO or PF.

## Patentansprüche

1. Elektrolytisches Lösungsmittel mit niedrigem Schmelzpunkt, dessen Kation vom Onium-Typ ist und wenigstens ein Heteroatom wie N, O, S oder P aufweist, das die positive Ladung trägt und dessen Anion ganz oder teilweise wenigstens ein Imidion vom Typ (FX¹O)N⁻(OX²)F umfasst, wobei X¹ und X² gleich oder verschieden voneinander sind und SO oder PF umfassen.
